# EUROPEAN PATENT APPLICATION

(11) **EP 3 919 468 A1**
(43) Date of publication of application: **08.12.2021**
(21) Application number: 20178014.5
(22) Date of filing: 03.06.2020
(51) Int. Cl.: C07C 41/06, C07C 41/42

(54) **SYSTEMS AND PROCESSES FOR PRODUCING METHYL TERTIARY BUTYL ETHER**

(71) Applicant: SABIC Global Technologies B.V., 4612 PX Bergen op Zoom (NL)
(72) Inventor: BADGANDI, Srikant Vasant, 562125 Bangalore (IN); HASYAGAR, Umesh Krishna, 562125 Bangalore (IN); NAIR, Vinod Sankaran, 562125 Bangalore (IN)
(74) Representative: J A Kemp LLP

(57) **Abstract**

System and process for producing methyl tertiary butyl ether (MTBE) is disclosed. The process can include contacting isobutylene feed with a first stream comprising methanol under conditions suitable to obtain a second stream comprising MTBE and unreacted isobutylene, separating the second stream to obtain a third stream comprising isobutylene and a products stream comprising MTBE, contacting the third stream with a fourth stream comprising methanol under conditions suitable to obtain an fifth stream comprising MTBE, and separating the fifth stream to obtain a sixth stream comprising MTBE. The isobutylene feed can be obtained from a C4 dehydrogenation unit and/or a raffinate-1 stream.

## Description

### CROSS REFERENCE TO RELATED APPLICATIONS

None.

### BACKGROUND OF THE INVENTION

### A. Field of the Invention

The invention generally concerns systems and processes for producing methyl tertiary butyl ether (MTBE). In one aspect, the invention can include systems and processes capable of producing MTBE using different C4 hydrocarbons feeds from different sources.

### B. Description of Related Art

Methyl tertiary butyl ether (MTBE) is an important petrochemical product with multiple industrial uses, such as gasoline booster compounds, solvents etc. MTBE is generally produced by a reaction of isobutylene and methanol. Industrially, isobutylene can be obtained from various sources such as from dehydrogenation of isobutane or from refining cracking products such as naphtha cracking products. An isobutylene feed, depending on its origin, e.g., process through which it was obtained, can have different isobutylene content and other hydrocarbons. Most of the current MTBE production plants typically use isobutylene from a single type of feed source, and major re-configuration of the plant is required if isobutylene from a different feed source is used. By way of example, International Application Publication WO2018022766A1 discloses a method for producing MTBE from a raffinate-1 stream. Parrafins are removed from a raffinate-1 stream to obtain an olefin rich stream and the olefin rich stream is reacted with methanol to produce MTBE. Systems and processes disclosed in WO2018022766 would need to be reconfigured if a non-raffinate-1 stream is used as the feed source, as other feed sources would have a different compositional make-up.

The time, expense, and technology that would be used to reconfigure an MTBE plant to accept a different feed source can become prohibitive. This results in reliance on a single isobutylene feed source for a given MTBE plant, which can be problematic if the isobutylene feed source is in limited supply and/or if the costs associated with such a feed source increases.

### SUMMARY OF THE INVENTION

A discovery has been made that provides a solution to at least some of the problems associated with MTBE production. In one aspect, the solution can include providing a process and system for MTBE production that is capable of using isobutylene feeds from multiple sources. In the systems and processes of the current invention, the operating conditions of the system units can remain similar irrespective of the isobutylene feed source. In certain aspects, the isobutylene feed used for MTBE production can be obtained from a C4 hydrocarbon dehydrogenation unit and/or from a raffinate-1 stream. An advantage of this is that reliance on a single isobutylene feed source may not be needed, which can be beneficial if, for example, one isobutylene feed source (e.g., C4 hydrocarbon dehydrogenation) is in limited supply and/or if the costs associated with such a feed source increases. In such instances, another feed source can be used (e.g., raffinate-1) while avoiding or reducing the time, expense, and/or technology that would be used to reconfigure the MTBE production plant to accept the new feed source.

One aspect of the present invention is directed to a process for producing MTBE. The process can include any one of, any combination of, or all of steps (a)-(d). In step (a) an isobutylene feed can be contacted with a first stream containing methanol under conditions suitable to obtain a second stream containing MTBE and unreacted isobutylene. In step (b) the second stream can be separated to obtain a third stream containing isobutylene and a products stream containing MTBE. In step (c) the third stream can be contacted with a fourth stream containing methanol under conditions suitable to obtain a fifth stream containing MTBE. In step (d) the fifth stream can be separated to obtain a sixth stream containing MTBE. In step (d) MTBE can be separated from C4 hydrocarbons, such as unreacted isobutylene, and C4 hydrocarbons other than isobutylene introduced in the process with the isobutylene feed.

The isobutylene feed in step (a) can be obtained from a C4 dehydrogenation unit or a raffinate-1 stream. In some aspects, a seventh stream containing isobutylene and isobutane from the C4 dehydrogenation unit can be separated to form an eight stream containing isobutylene and a ninth stream containing isobutane, and the eight stream can be fed to, e.g., contacted with, the first stream, in step (a) forming the isobutylene feed of step (a). In some aspects, the seventh stream can contain 30 wt. % to 50 wt. % of isobutane and/or 30 wt. % to 50 wt. % of isobutylene. The eight stream can contain 60 wt. % to 95 wt. % isobutylene and/or 5 wt. % to 30 wt. % of isobutane. The ninth stream can contain 1 wt. % to 35 wt. % of isobutylene and/or 55 wt. % to 99 wt. % of isobutane. In some aspects, the ninth stream can be recycled to the dehydrogenation unit. The seventh stream can be separated in a C4 separation unit. The C4 separation unit can include a distillation column and the column operation condition during the separation process can include a column top temperature 45 °C to 50 °C, a column bottom temperature 52 °C to 60 °C, a pressure 6 to 9 bar, or a reflux ratio 10 to 30 or any combination thereof. The distillation column can include 50 to 200 trays. Enrichment of isobutylene via the C4 separation unit can decrease the circulation of inert components and thereby reduce the associated downstream equipment size of the MTBE production process.

In some other aspects, the isobutylene feed in step (a) can be obtained from a raffinate-1 stream, where a raffinate-1 stream can be fed to, e.g. contacted with the first stream, in step (a) forming the isobutylene feed of step (a). In some aspects, the raffinate-1 stream can contain 30 wt.% to 50 wt.% of isobutylene, 5 wt.% to 10 wt.% of isobutane, 15 wt.% to 30 wt.% of 1-butene, 0 wt.% to 10 wt.% of trans-butene, 0 wt.% to 10 wt.% of n-butane, 0 wt.% to 5 wt.% of cis-butene, and/or 0 to 5 wt.% of 1, 3 butadiene or any combination thereof. In some aspects, the raffinate-1 stream can be obtained from refining a product stream obtained from a cracking process such as a naphtha cracking process.

In step (a) and (c) MTBE can be produced by a reaction of isobutylene and methanol, and the contacting condition, e.g., reaction condition of isobutylene and methanol, in step (a) and/or (c) can include a pressure of 10 bar to 25 bar, preferably 15 bar to 20 bar and/or a temperature of 35 °C to 90 °C preferably 40 °C to 70 °C. In step (a) and/or (c) the methanol can be reacted with the isobutylene at a molar ratio of 1:1 to 1.1:1. In some aspects, the reaction of isobutylene and methanol can be catalyzed by an etherification catalyst. In some aspects, the etherification catalyst can be an acidic cationic polymeric catalyst. In some aspects, the etherification catalyst can be an acidic cationic polymeric catalyst with a polymer matrix and acidic functional groups. In some aspects, the etherification catalyst can be an acidic cationic polymeric catalyst with macroporous polystyrene matrix and sulfonic acid functional groups. In some aspects, isobutylene conversion in step (a) and/or step (c) can be 70 to 95%. In some aspects, the step (a) can be performed in a first reactor and the step (c) can be performed in a second reactor, and the volume e.g. inner volume of the second reactor can be less than the volume e.g. inner volume of the first reactor. In some aspects, a ratio of inner volume of the second reactor and the inner volume of the first reactor can be 0.6:1 to 0.8:1. Reducing the size of the second reactor can reduce the capital investment and/or make the plant operation relatively easier, making the systems and processes of the current invention more efficient than systems and processes where unreacted isobutylene from an MTBE reactor is recycled to the same reactor.

In some aspects, in step (b) the second stream can be separated in a first MTBE separation unit. In some aspects, the first MTBE separation unit can include a fractionation column and the fractionation column operating condition during the separation process can include a column top temperature of 45 °C to 60 °C, a column bottom temperature of 100 °C to 125 °C, a pressure of 4 to 7 bar, or a reflux ratio of 1 to 2 or any combination thereof. The fractionation column of the first MTBE separation unit can include 10 to 20 trays. The products stream obtained in step (b) can include 95 wt. % to 100 wt. %, preferably 96 wt. % to 100 wt. %, more preferably 98 wt. % to 100 wt. % of MTBE. In some aspects, in step (d) the fifth stream can be separated in a second MTBE separation unit. In some aspects, the second MTBE separation unit can include a fractionation column and the fractionation column operating condition during the separation process can include a column top temperature of 45 °C to 60 °C, a column bottom temperature of 100 °C to 125 °C, a pressure of 4 to 7 bar, or a reflux ratio of 1 to 5 or any combination thereof. The fractionation column of the second MTBE separation unit can include 10 to 20 trays. In some particular aspects, the process can further include feeding the sixth stream from the second separation unit to the first separation unit. The second reactor and the second MTBE separation unit can produce additional MTBE from the unreacted iso-butylene coming out of the first reactor and the first MTBE separation. The introduction of the second downstream reactor not only improves the yield of MTBE product but also eliminates the need for further recycling. This means that since there are no other undesired products present, the size of the second reactor can be much smaller than the first reactor. This also means that a smaller distillation column is needed to purify MTBE from the second reactor as compared to the upstream distillation column.

In step (d) the fifth stream can be separated to form the sixth stream, and a tenth stream or an eleventh stream. A tenth stream containing isobutane can be formed from the step (d) if the isobutylene feed of step (a) is obtained from the C4 dehydrogenation unit i.e., the eighth stream is fed to step (a). At least a portion of the isobutane in the tenth stream can be from the eight stream. An eleventh stream containing n-butane, isobutane, 1-butene, cis-butene, trans-butene, or 1, 3 butadiene or any combination thereof can be formed from step (d) if the isobutylene feed of step (a) is obtained from the raffinate-1 stream i.e., raffinate-1 stream is fed to step (a). At least a portion of the C4 hydrocarbons in the eleventh stream can be from the raffinate-1 stream. In some aspects, the process can further include separating the eleventh stream to obtain a twelfth stream containing iso-butane and/or n-butane and a thirteenth stream containing 1-butene, cis-butene, trans-butene, and/or 1, 3 butadiene. In some aspects, the thirteenth stream can be further separated to form a fourteenth stream containing 1-butene and a fifteenth stream containing cis-butene, trans-butene, and/or 1, 3 butadiene. In some aspects, the eleventh stream can be separated in a C4 separation unit. The C4 separation unit can include a distillation column and the column operation condition during the separation process can include a column top temperature of 45 °C to 50 °C, a column bottom temperature of 50 °C to 60 °C, a pressure of 6 to 9 bar, or a reflux ratio of 10 to 30 or any combination thereof. The distillation column can include 50 to 200 trays. In some aspects, the thirteenth stream can be separated with a butene separation column.

Another aspect of the present invention is directed to a system for producing MTBE. The system can include a first reactor, a first MTBE separation unit, a second reactor, and a second MTBE separation unit. The first reactor can be configured to receive an isobutylene feed and a first stream containing methanol, and produce a second stream containing MTBE and unreacted isobutylene by a reaction of methanol and isobutylene. The first MTBE separation unit can be operatively connected to the first reactor and arranged downstream to the first reactor, configured to receive the second stream, separate MTBE and isobutylene, and produce a products stream containing MTBE and a third stream containing isobutylene. The second reactor can be operatively connected to the first MTBE separation unit and arranged downstream to the first MTBE separation unit, configured to receive the third stream and a fourth stream containing methanol and produce a fifth stream containing MTBE by a reaction of methanol and isobutylene. The second MTBE separation unit can be operatively connected to the second reactor and arranged downstream to the second reactor, configured to receive the fifth stream, separate MTBE from C4 hydrocarbons, and produce a sixth stream comprising MTBE. In some aspects, the volume e.g. inner volume of the first reactor can be larger than the volume, e.g., inner volume of the second reactor.

The system can further include a C4 separation unit configured to separate C4 hydrocarbons. The C4 separation unit can be operatively connected to the first reactor or the second MTBE separation unit depending on the isobutylene feed source to the first reactor. In some aspects, the system can further include a C4 dehydrogenation unit and the isobutylene feed to the first reactor can be obtained from the C4 dehydrogenation unit. The C4 separation unit can be operatively connected to the first reactor and the C4 dehydrogenation unit. The C4 separation unit can be configured to receive a seventh stream containing isobutylene and isobutane from the C4 dehydrogenation unit, separate isobutylene and isobutane to produce an eighth stream containing isobutylene and a ninth stream containing isobutane. The eighth stream containing isobutylene can be fed to the first reactor, forming the isobutylene feed for the first reactor.

In some aspects, the first reactor can be configured to receive the isobutylene feed from a raffinate-1 stream and the C4 separation unit can be operatively connected to the second MTBE separation unit. If the raffinate-1 stream is fed to the first reactor, in the second MTBE separation unit the fifth stream can be separated into the sixth stream and an eleventh stream containing n-butane, iso-butane, 1-butene, cis-butene, trans-butene, or 1, 3-butadiene, or a combination thereof, wherein at least a portion of the C4 hydrocarbons in the eleventh stream can be from the raffinate-1 stream. The C4 separation unit can be configured to receive the eleventh stream from the second MTBE separation unit, and separate the eleventh stream to form a twelfth stream containing n-butane and/or iso-butane and a thirteenth stream containing 1-butene, cis-butene, trans-butene, or 1, 3-butadiene any combination thereof. In some aspects, the system can further include a butene separation column, and the butene separation column can be configured to receive the thirteenth stream and separate the thirteenth stream to produce a fourteenth stream containing 1-butene and a fifteen stream containing cis-butene, trans-butene, and/or 1, 3-butadiene.

Other embodiments of the invention are discussed throughout this application. Any embodiment discussed with respect to one aspect of the invention applies to other aspects of the invention as well and vice versa. Each embodiment described herein is understood to be embodiments of the invention that are applicable to other aspects of the invention. It is contemplated that any embodiment discussed herein can be implemented with respect to any method or composition of the invention, and vice versa. Furthermore, compositions and systems of the invention can be used to achieve methods of the invention.

The following includes definitions of various terms and phrases used throughout this specification.

C4 hydrocarbons refers to hydrocarbons with molecules having 4 carbon atoms, such as n-butane, iso-butane, 1-butene, cis-butene, trans-butene, and 1, 3 butadiene. A C4 hydrocarbon stream can include a stream that comprises, consists essentially of, or consists of C4 hydrocarbons. A C4 separation unit can be used to separate or isolate targeted or desired C4 hydrocarbons (e.g., isobutylene and/or isobutane) from a C4 hydrocarbon stream.

The terms "about" or "approximately" are defined as being close to as understood by one of ordinary skill in the art. In one non-limiting embodiment, the terms are defined to be within 10%, preferably within 5%, more preferably within 1%, and most preferably within 0.5%.

The terms "wt.%," "vol.%," or "mol.%" refers to a weight percentage of a component, a volume percentage of a component, or molar percentage of a component, respectively, based on the total weight, the total volume of material, or total moles, that includes the component. In a non-limiting example, 10 grams of component in 100 grams of the material is 10 wt.% of component.

The term "substantially" and its variations are defined to include ranges within 10%, within 5%, within 1%, or within 0.5%.

The terms "inhibiting" or "reducing" or "preventing" or "avoiding" or any variation of these terms, when used in the claims and/or the specification includes any measurable decrease or complete inhibition to achieve a desired result.

The term "effective," as that term is used in the specification and/or claims, means adequate to accomplish a desired, expected, or intended result.

The use of the words "a" or "an" when used in conjunction with any of the terms "comprising," "including," "containing," or "having" in the claims, or the specification, may mean "one," but it is also consistent with the meaning of "one or more," "at least one," and "one or more than one."

The phrase "and/or" means and or or. To illustrate, A, B, and/or C includes: A alone, B alone, C alone, a combination of A and B, a combination of A and C, a combination of B and C, or a combination of A, B, and C. In other words, "and/or" operates as an inclusive or.

The words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The process and systems of the present invention can "comprise," "consist essentially of," or "consist of" particular ingredients, components, compositions, steps, *etc.* disclosed throughout the specification. With respect to the transitional phrase "consisting essentially of," in one non-limiting aspect, a basic and novel characteristic of the processes and the systems of the present invention can be their abilities to produce MTBE from different isobutylene feed sources (e.g., a C4 dehydrogenation feed or a raffinate-1 feed).

Other objects, features and advantages of the present invention will become apparent from the following figures, detailed description, and examples. It should be understood, however, that the figures, detailed description, and examples, while indicating specific embodiments of the invention, are given by way of illustration only and are not meant to be limiting. Additionally, it is contemplated that changes and modifications within the spirit and scope of the invention will become apparent to those skilled in the art from this detailed description. In further embodiments, features from specific embodiments may be combined with features from other embodiments. For example, features from one embodiment may be combined with features from any of the other embodiments. In further embodiments, additional features may be added to the specific embodiments described herein.

### BRIEF DESCRIPTION OF THE DRAWINGS

Advantages of the present invention may become apparent to those skilled in the art with the benefit of the following detailed description and upon reference to the accompanying drawings.
**FIG. 1** is a schematic of one example of the present invention to produce MTBE.
**FIG. 2** is a schematic of an example of the present invention to produce MTBE using system of FIG. 1.
**FIG. 3** is a schematic of an another example of the present invention to produce MTBE using system of FIG. 1.

While the invention is susceptible to various modifications and alternative forms, specific embodiments thereof are shown by way of example in the drawings. The drawings may not be to scale.

### DETAILED DESCRIPTION OF THE INVENTION

A discovery has been made that provides a solution to at least some of the aforementioned problems associated with MTBE production. In one aspect, the solution can include providing MTBE production processes and systems that are flexible enough to use isobutylene feed from different feed sources, without changing, or reducing or limiting the changes of, the overall operating conditions of certain system units. The MTBE production processes and systems of the current invention can result in relatively simplified plant configuration irrespective of isobutylene feed source, reduced plant size, efficient utilization of floor space, and/or enhanced equipment performance as compared to current MTBE production plants. In certain aspects, along with MTBE other valuable petrochemical products such as relatively purified streams of 1-butene, isobutane can be obtained from the processes and systems of the current invention.

These and other non-limiting aspects of the present invention are discussed in further detail in the following paragraphs with reference to the figures.

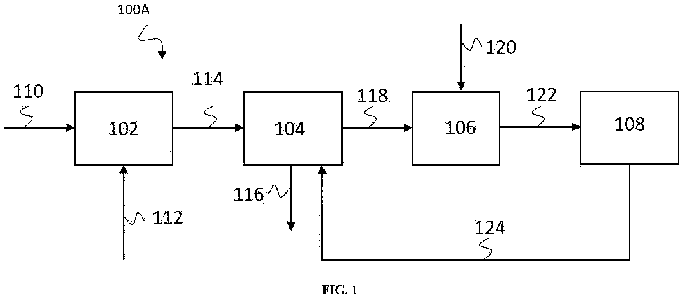

Referring to FIG. 1, one example of the system and process of the present invention for producing MTBE is described. System 100A can include a first reactor 102, a first MTBE separation unit 104, a second reactor 106, and a second MTBE separation unit 108. A stream 110 containing isobutylene feed and a first stream 112 containing methanol can be fed to the first reactor 102. The stream 110 and the first stream 112 can be fed to the first reactor 102 as separate feeds or the streams 110 and 112 can be combined before and fed to the first reactor 102 as a single feed (not shown). The isobutylene and the alcohol can react in the first reactor 102 under conditions sufficient to produce a second stream 114 containing MTBE and unreacted isobutylene. The first reactor 102 can contain an etherification catalyst (not shown) and the reaction of isobutylene and methanol can be catalyzed by the etherification catalyst. The second stream 114 can be fed to the first MTBE separation unit 104. In the first MTBE separation unit 104 the second stream 114 can be separated into a products stream 116 containing at least a portion of the MTBE and a third stream 118 containing at least a portion of the unreacted isobutylene. The third stream 118 and a fourth stream 120 containing methanol can be fed to the second reactor 106. The stream 118 and the stream 120 can be fed to the second reactor 106 as separate feeds or the streams 118 and 120 can be combined before and fed to the second reactor 106 as a single feed (not shown). The isobutylene and methanol can react in the second reactor 106 under conditions sufficient to produce a fifth stream 122 containing MTBE. The second reactor 106 can contain an etherification catalyst (not shown) and the reaction of the isobutylene and the methanol can be catalyzed by the etherification catalyst. The fifth stream 122 can be fed to the second MTBE separation unit 108. In the second MTBE separation unit 108 the fifth stream 122 can be separated into a sixth stream 124 containing at least a portion of the MTBE, and subsequent other streams (not shown). The sixth stream 124 from the second MTBE separation unit 108 can be recycled to the first MTBE separation unit 104. Alternatively, the sixth stream 124 from the second MTBE separation unit 108 can be isolated for further downstream processing or use (not shown).

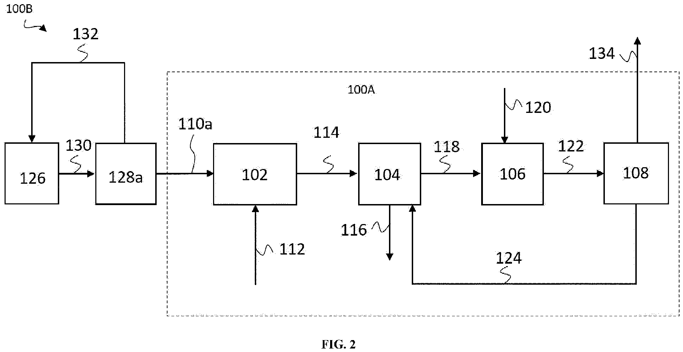

Referring to FIG. 2, another example of the system and process of the present invention for producing MTBE is described. For the system and process of FIG. 2, the isobutylene feed can be obtained from a C4 dehydrogenation unit. The system 100B can include a C4 dehydrogenation unit 126, a C4 separation unit 128a and the system 100A (see FIG. 1). For system 100B, the isobutylene feed (stream 110 in FIG. 1), referred to as stream 110a in FIG. 2, can be obtained from the dehydrogenation unit 126 via the C4 separation unit 128a. A seventh stream 130 containing isobutylene and isobutane can be fed to the C4 separation unit 128a from the C4 dehydrogenation unit 126. In the C4 separation unit 128a the seventh stream can be separated to form an eight stream 110a containing isobutylene and a ninth stream 132 containing isobutane. The eight stream 110a can be fed to the first MTBE reactor 102 of system 100A forming the isobutylene feed stream (stream 110 in FIG. 1) of system 100A. The eight stream can further include isobutane. At least a portion of the isobutane from the eight stream 110a can be transferred to the second separation unit 108, via streams 114, 118 and 122. In the second separation unit 108 the stream 122 can be separated to form the sixth stream 124 and a tenth stream 134 containing isobutane. In some aspects, the stream 134 can be used in further process steps as a raffinate-2 stream (not shown).

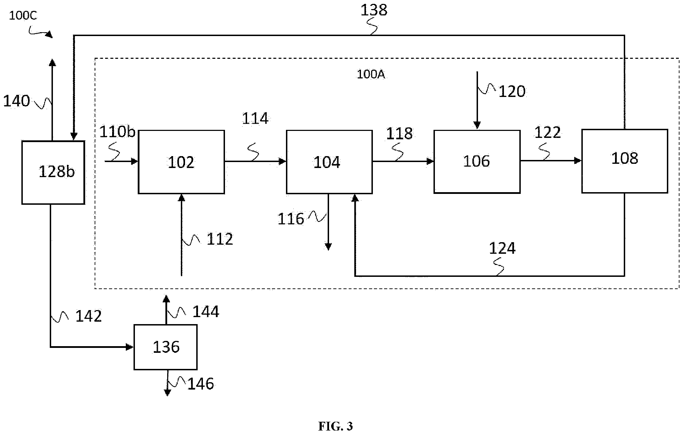

Referring to FIG. 3, another example of the system and process of the present invention for producing MTBE is described. For system 100C, the isobutylene feed (stream 110 in FIG. 1), referred to as stream 110b in FIG. 3, can be a raffinate-1 stream. The system 100C can include, system 100A, a C4 separation unit 128b, and a butene separation column 136. The raffinate-1 stream 110b containing isobutylene can be fed to the first MTBE reactor 102 of system 100A forming the isobutylene feed stream (stream 110 in FIG. 1) of system 100A. The raffinate-1 stream can further include C4 hydrocarbons other than isobutylene such as isobutane, n-butane, 1-butene, cis-butene, trans-butene, 1, 3, butadiene or any combination thereof. At least a portion of the C4 hydrocarbons from the raffinate-1 stream 110b can be transferred to the second separation unit 108, via streams 114, 118, and 122. In the second separation unit 108, the stream 122 can be separated to form the sixth stream 124 and a eleventh stream 138 containing n-butane, iso-butane, 1-butene, cis-butene, trans-butene, 1, 3, butadiene or any combination thereof. The eleventh stream 138 can be fed to the C4 separation unit 128b. In the C4 separation unit the eleventh stream 138 can get separated to form a twelfth stream 140 containing butanes, such as n-butane and/or iso-butane and a thirteenth stream 142 containing butenes such as 1-butene, cis-butene, trans-butene, 1, 3, butadiene or any combination thereof. The thirteenth stream 142 can be fed to the butene separation column 136, and can get separated to form a fourteenth stream 144 containing 1-butene and a fifteen stream 146 containing cis-butene, trans-butene, 1, 3, butadiene. In some aspects, the stream 142 or a portion of the stream 142, can be mixed with the stream 140 or a portion of the stream 140, and can be used in further process steps as a raffinate-2 stream (not shown).

Referring to any one of FIGS. 1-3, in the first reactor 102 and/or the second reactor 106, MTBE can be produced by an etherification reaction between isobutylene and methanol. The reaction conditions in the first reactor 102 and/or the second reactor 106 can include: (i) a temperature of 35 °C to 90 °C or at least any one of, equal to any one of, or between any two of 35 °C, 40 °C, 45 °C, 50 °C, 55 °C, 60 °C, 65 °C, 70 °C, 75 °C, 80 °C, 85 °C, and 90 °C; and/or (ii) a pressure of 10 bar to 25 bar or at least any one of, equal to any one of, or between any two of 10 bar, 11 bar, 12 bar, 13 bar, 14 bar, 15 bar, 16 bar, 17 bar, 18 bar, 19 bar, 20 bar, 21 bar, 22 bar, 23 bar 24 bar, and 25 bar. Methanol and isobutylene can be fed to the first reactor 102 and/or the second reactor 106 at a molar ratio of 1:1 to 1.1:1 or at least any one of, equal to any one of, or between any two of 1:1, 1.02:1, 1.04:1, 1.06:1, 1.08:1 and 1.1:1. The isobutylene conversion for the etherification reaction in the first reactor 102 and/or the second reactor 106 can be 70 % to 95 % or at least any one of, equal to any one of, or between any two of 70 %, 71 %, 72 %, 73 %, 74 %, 75 %, 76 %, 77 %, 78 %, 79 %, 80 %, 81 %, 82 %, 83 %, 84 %, 85 %, 86 %, 87 %, 88 %, 89 %, 90 %, 91 %, 92 %, 93 %, 94 %, and 95 %. The reactors 102 and/or 106 can contain an etherification catalyst and the etherification catalyst can catalyze the reaction between isobutylene and methanol. The etherification catalyst can be an etherification catalyst known in the art. In some aspects, the etherification catalyst can be an acidic cationic polymeric catalyst. In some aspects, the etherification catalyst can be an acidic cationic polymeric catalyst with a polymer matrix and acidic functional groups. In some aspects, the etherification catalyst can be an acidic cationic polymeric catalyst with macroporous polystyrene matrix and sulfonic acid functional groups. In some particular aspects, the etherification catalyst can be DOW AMBERLYST 15 (DowDuPont (Midland, Michigan)) which is an acidic cationic polymeric catalyst with macroporous styrene-divenylbenzene matrix and sulfonic acid functional groups; DOW AMBERLYST 35 (DowDuPont (Midland, Michigan)) which is an acidic cationic polymeric catalyst with macroporous styrene-divenylbenzene matrix and sulfonic acid functional groups; DOW CSP-3 (DowDuPont (Midland, Michigan)) which is an acidic cationic polymeric catalyst with macroporous styrene-divenylbenzene matrix and sulfonic acid functional groups; PUROLITE CT-175 (Purolite (Bala Cynwyd, PA)) which is an acidic cationic polymeric catalyst with macroporous styrene-divenylbenzene matrix and sulfonic acid functional groups; PUROLITE CT-275 (Purolite (Bala Cynwyd, PA)) which is an acidic cationic polymeric catalyst with macroporous styrene-divenylbenzene matrix and sulfonic acid functional groups; LANXESS K-2620 (Lenntech (Delfgauw, Netherlands)) which is an acidic cationic polymeric catalyst with macroporous crosslinked polystyrene matrix and sulfonic acid functional groups; LANXESS K-2629 (Lenntech (Delfgauw, Netherlands)) which is an acidic cationic polymeric catalyst with macroporous crosslinked polystyrene matrix and sulfonic acid functional groups; or SINOCATA S-600 (Sino Catalyst Co Limited, Hong Kong)) which is an acidic cationic polymeric catalyst with macroporous crosslinked polystyrene matrix and sulfonic acid functional groups; or any combination thereof. In some embodiments, a wet etherification catalyst can be loaded in the reactors 102 and/or 106 and methanol can be used to remove moisture/water from the catalyst. The etherification catalyst in the first reactor 102 can be same or different than the etherification catalyst in the second reactor 106. The volume e.g. inner volume of the reactor 106 can be smaller than the volume e.g. inner volume of the reactor 102. In some aspects, the ratio of the inner volume of reactor 106 and 102 can be 0.6:1 to 0.8:1 or at least any one of, equal to any one of, or between any two 0.6:1, 0.65:1, 0.7:1, 0.75:1 and 0.8:1.

In the 1st MTBE separation unit 104, MTBE can be separated from C4 hydrocarbons by any suitable methods known in the art, e.g., distillation, fractionation, pressure swing adsorption, and the like. In some aspects, the separation unit 104 can contain a fractionation column. In some particular aspects, the unit 104 fractionation column operating conditions during MTBE separation process can include: i) a column top temperature of 45 °C to 60 °C, or at least any one of, equal to any one of, or between any two of 45 °C, 46 °C, 48 °C, 50 °C, 52 °C, 54°C, 56 °C, 58 °C and 60 °C; ii) a column bottom temperature of 100 °C to 125 °C, or at least any one of, equal to any one of, or between any two of 100 °C, 105 °C, 110 °C, 115 °C, 120 °C, and 125 °C; iii) a pressure of 4 bar to 7 bar or at least any one of, equal to any one of, or between any two of 4 bar, 4.5 bar 5 bar, 5.5 bar 6 bar, 6.5 bar, and 7 bar, or (iv) a reflux ratio of 1 to 5 or 1 to 2 or at least any one of, equal to any one of, or between any two of 1, 1.5, 2, 3, 4 and 5, or any combination thereof. The column can include 8 to 25 or 10 to 20 or at least any one of, equal to any one of, or between any two of 8, 9, 10, 11, 12, 13, 14, 15, 16, 17, 18, 19, 20, 21, 22, 23, 24 and 25 trays. The fractionation column of the 1st MTBE separation unit can produce the third stream as the top product and MTBE containing products stream as the bottom product.

In the 2nd MTBE separation unit 108, MTBE can be separated from the C4 hydrocarbons by any suitable methods known in the art, e.g., distillation, fractionation, pressure swing adsorption, and the like. In some aspects, the separation unit 108 can contain a fractionation column. In some particular aspects, the unit 108 fractionation column operating conditions during MTBE separation process can include: i) a column top temperature of 45 °C to 60 °C, or at least any one of, equal to any one of, or between any two of 45 °C, 46 °C, 48 °C, 50 °C, 52 °C, 54°C, 56 °C, 58 °C and 60 °C; ii) a column bottom temperature of 100 °C to 125 °C, or at least any one of, equal to any one of, or between any two of 100 °C, 105 °C, 110 °C, 115 °C, 120 °C, and 125 °C; iii) a pressure of 4 bar to 7 bar at least any one of, equal to any one of, or between any two of 4 bar, 4.5 bar 5 bar, 5.5 bar 6 bar, 6.5 bar, and 7 bar, or (iv) a reflux ratio of 1 to 5 or at least any one of, equal to any one of, or between any two of 1, 1.5, 2, 3, 4 and 5, or any combination thereof. The column can include 8 to 25 or 10 to 20 or at least any one of, equal to any one of, or between any two of 8, 9, 10, 11, 12, 13, 14, 15, 16, 17, 18, 19, 20, 21, 22, 23, 24 and 25 trays. The fractionation column of the 2nd MTBE separation unit can produce the MTBE containing sixth stream 124 as the bottom product. The top product of the 2nd MTBE separation unit can be a C4 hydrocarbons containing stream and the composition of the C4 hydrocarbons containing stream can depend on the isobutylene feed to the first reactor 102. Referring FIG. 2 when the isobutylene feed is obtained from a dehydrogenation unit, the C4 hydrocarbons stream, stream 134 of FIG. 2, from the 2nd MTBE separation unit can contain isobutane. In some aspects, the stream 134 can contain 75 wt. % to 99 wt. % or 95 wt. % to 99 wt. % of isobutane. At least a portion of the isobutane in the stream 134 can come from the eight stream 110a i.e. isobutylene feed stream to the first reactor. Referring FIG. 3 when the isobutylene feed is obtained from a raffinate-1 stream, the C4 hydrocarbons stream, stream 138 of FIG. 3, from the 2nd MTBE separation unit can contain isobutane, 1-butene, cis-butane, trans-butane, or 1,3 butadiene or any combination thereof. At least a portion of the C4 hydrocarbons in the stream 138 can come from the raffinate-1 stream 110b i.e. isobutylene feed stream to the first reactor.

The C4 separation unit 128a and 128b, can separate C4 hydrocarbons such as butanes (e.g. n-butane and/or iso-butane) from butenes (isobutylene, 1-butene, cis-butene, trans-butene and/or 1,3-butadiene). The separation of butenes and butanes in the C4 separation unit 128a and/or 128b can be obtained by any suitable methods known in the art e.g., distillation, fractionation, pressure swing adsorption, and the like. In some aspects, the C4 separation unit 128a and/or 128b, can contain a distillation column. In some particular aspects, the distillation column operating conditions during the separation process can include i) a column top temperature of 45 °C to 50 °C, or at least any one of, equal to any one of, or between any two of 45 °C, 46 °C, 47 °C, 48 °C, 49 °C, and 50 °C; ii) a column bottom temperature of 50 °C to 60 °C, or 52 °C to 60 °C,or at least any one of, equal to any one of, or between any two of 50 °C, 52 °C, 54 °C, 56 °C, 58 °C, and 60 °C; iii) a pressure of 6 bar to 9 bar at least any one of, equal to any one of, or between any two of 6 bar, 6.5 bar, 7 bar, 7.5 bar 8 bar, 8.5 bar and 9 bar, or (iv) a reflux ratio of 10 to 30, or at least any one of, equal to any one of, or between any two of 10, 15, 20, 25 and 30, or any combination thereof. The column can include 50 to 200 or at least any one of, equal to any one of, or between any two of 50, 60, 80, 100, 120, 140, 160, 180, and 200 trays.

In the C4 dehydrogenation unit 126, isobutylene can be produced by dehydrogenation of isobutane. The dehydrogenation reaction conditions can include i) a temperature of 560 °C to 700 °C or at least any one of, equal to any one of, or between any two of 560 °C, 570 °C, 580 °C, 590 °C, 600 °C, 610 °C, 620 °C, 630 °C, 640 °C, 650 °C, 660 °C, 670 °C, 680 °C, 690 °C, and 700 °C; and/or ii) a pressure of 0.1 bar to 0.6 bar or at least any one of, equal to any one of, or between any two of 0.1 bar, 0.2 bar, 0.3 bar, 0.4 bar, 0.5 bar and 0.6 bar. In some aspects, the dehydrogenation of isobutane can be catalyzed by a dehydrogenation catalyst. The dehydrogenation catalyst can be an isobutane dehydrogenation catalyst known in the art. In some aspects, the dehydrogenation catalyst can be a chromium oxide containing catalyst, platinum containing catalyst, platinum-tin containing catalyst, or any combination thereof.

The butene separation column 136 can separate 1-butene from other butene isomers such as cis-butene, trans-butene and/or 1,3-butadiene. The butene separation column 136 can be a butene separation column known in the art. The stream 144 obtained from the butene separation column 136 can include 95 wt. % to 100 wt. % or 97 wt. % to 99 wt. % of 1-butene.

The seventh stream 130 can include i) 30 wt.% to 50 wt.% or at least any one of, equal to any one of, or between any two of 30 wt.%, 31 wt.%, 32 wt.%, 33 wt.%, 34 wt.%, 35 wt.%, 36 wt.%, 37 wt.%, 38 wt.%, 39 wt.%, 40 wt.%, 41 wt.%, 42 wt.%, 43 wt.%, 44 wt.%, 45 wt.%, 46 wt.%, 47 wt.%, 48 wt.%, 49 wt.%, and 50 wt.% of isobutane; and/or ii) 30 wt.% to 50 wt.% or at least any one of, equal to any one of, or between any two of 30 wt.%, 31 wt.%, 32 wt.%, 33 wt.%, 34 wt.%, 35 wt.%, 36 wt.%, 37 wt.%, 38 wt.%, 39 wt.%, 40 wt.%, 41 wt.%, 42 wt.%, 43 wt.%, 44 wt.%, 45 wt.%, 46 wt.%, 47 wt.%, 48 wt.%, 49 wt.%, and 50 wt.% of isobutylene. In some aspects, the seventh stream 130 can further contain 0 to 15 wt.% of C4 hydrocarbons other than isobutane and isobutylene.

The eight stream 110a can include i) 5 wt.% to 30 wt.% or at least any one of, equal to any one of, or between any two of 5 wt.%, 6 wt.%, 7 wt.%, 8 wt.%, 9 wt.%, 10 wt.%, 11 wt.%, 12 wt.%, 13 wt.%, 14 wt.%, 15 wt.%, 16 wt.%, 17 wt.%, 18 wt.%, 19 wt.%, 20 wt.%, 21 wt.%, 22 wt.%, 23 wt.%, 24 wt.%, 25 wt.%, 26 wt.%, 27 wt.%, 28 wt.%, 29 wt.%, and 30 wt.% of isobutane; and/or ii) 60 wt.% to 95 wt.% or at least any one of, equal to any one of, or between any two of 60 wt.%, 61 wt.%, 62 wt.%, 63 wt.%, 64 wt.%, 65 wt.%, 66 wt.%, 67 wt.%, 68 wt.%, 69 wt.%, 70 wt.%, 71 wt.%, 72 wt.%, 73 wt.%, 74 wt.%, 75 wt.%, 76 wt.%, 77 wt.%, 78 wt.%, 79 wt.%, 80 wt.%, 81 wt.%, 82 wt.%, 83 wt.%, 84 wt.%, 85 wt.%, 86 wt.%, 87 wt.%, 88 wt.%, 89 wt.%, 90 wt.%, 91 wt.%, 92 wt.%, 93 wt.%, 94 wt.%, and 95 wt.% of isobutylene.

The ninth stream 132 can include i) 1 wt.% to 35 wt.% or at least any one of, equal to any one of, or between any two of 1 wt.%, 2 wt.%, 3 wt.%, 4 wt.%, 5 wt.%, 6 wt.%, 7 wt.%, 8 wt.%, 9 wt.%, 10 wt.%, 11 wt.%, 12 wt.%, 13 wt.%, 14 wt.%, 15 wt.%, 16 wt.%, 17 wt.%, 18 wt.%, 19 wt.%, 20 wt.%, 21 wt.%, 22 wt.%, 23 wt.%, 24 wt.%, 25 wt.%, 26 wt.%, 27 wt.%, 28 wt.%, 29 wt.%, 30 wt.%, 31 wt.%, 32 wt.%, 33 wt.%, 34 wt.%, and 35 wt.% of isobutylene; and/or ii) 55 wt.% to 99 wt.% or at least any one of, equal to any one of, or between any two of 55 wt.%, 56 wt.%, 57 wt.%, 58 wt.%, 59 wt.%, 60 wt.%, 61 wt.%, 62 wt.%, 63 wt.%, 64 wt.%, 65 wt.%, 66 wt.%, 67 wt.%, 68 wt.%, 69 wt.%, 70 wt.%, 71 wt.%, 72 wt.%, 73 wt.%, 74 wt.%, 75 wt.%, 76 wt.%, 77 wt.%, 78 wt.%, 79 wt.%, 80 wt.%, 81 wt.%, 82 wt.%, 83 wt.%, 84 wt.%, 85 wt.%, 86 wt.%, 87 wt.%, 88 wt.%, 89 wt.%, 90 wt.%, 91 wt.%, 92 wt.%, 93 wt.%, 94 wt.%, 95 wt.%, 96 wt.%, 97 wt.%, 98 wt.%, and 99 wt.% of isobutane.

The raffinate-1 stream 110b can include i) 30 wt.% to 50 wt.% or at least any one of, equal to any one of, or between any two of 30 wt.%, 31 wt.%, 32 wt.%, 33 wt.%, 34 wt.%, 35 wt.%, 36 wt.%, 37 wt.%, 38 wt.%, 39 wt.%, 40 wt.%, 41 wt.%, 42 wt.%, 43 wt.%, 44 wt.%, 45 wt.%, 46 wt.%, 47 wt.%, 48 wt.%, 49 wt.%, and 50 wt.% of isobutylene; ii) 5 wt.% to 10 wt.% or at least any one of, equal to any one of, or between any two of 5 wt.%, 6 wt.%, 7 wt.%, 8 wt.%, 9 wt.%, and 10 wt.% of isobutane; iii) 15 wt.% to 30 wt.% or at least any one of, equal to any one of, or between any two of 15 wt.%, 16 wt.%, 17 wt.%, 18 wt.%, 19 wt.%, 20 wt.%, 21 wt.%, 22 wt.%, 23 wt.%, 24 wt.%, 25 wt.%, 26 wt.%, 27 wt.%, 28 wt.%, 29 wt.%, and 30 wt.%, of 1-butene, iv) 0 to 10 wt.% or at least any one of, equal to any one of, or between any two of 0, 0.1 wt.%, 1 wt.%, 2 wt.%, 3 wt.%, 4 wt.% 5 wt.%, 6 wt.%, 7 wt.%, 8 wt.%, 9 wt.%, and 10 wt.% of trans-butene; v) 0 to 10 wt.% or at least any one of, equal to any one of, or between any two of 0, 0.1 wt.%, 1 wt.%, 2 wt.%, 3 wt.%, 4 wt.% 5 wt.%, 6 wt.%, 7 wt.%, 8 wt.%, 9 wt.%, and 10 wt.% of n-butane; vi) 0 to 5 wt.% or at least any one of, equal to any one of, or between any two of 0, 0.1 wt.%, 1 wt.%, 2 wt.%, 3 wt.%, 4 wt.% and 5 wt.%, 6 wt.%, 7 wt.%, 8 wt.%, 9 wt.%, and 10 wt.% of cis-butene; or vi) 0 to 5 wt.% or at least any one of, equal to any one of, or between any two of 0, 0.1 wt.%, 1 wt.%, 2 wt.%, 3 wt.%, 4 wt.% and 5 wt.% of 1, 3 butadiene or any combination thereof.

The first stream 112 and/or the fourth stream 120 can include 90 wt.% to 100 wt.% or at least any one of, equal to any one of, or between any two of 90 wt.%, 91 wt.%, 92 wt.%, 93 wt.%, 94 wt.%, 95 wt.%, 96 wt.%, 97 wt.%, 98 wt.%, 98 wt.%, and 100 wt.% of methanol.

The products stream 116 can include 90 wt.% to 100 wt.% or at least any one of, equal to any one of, or between any two of 90 wt.%, 91 wt.%, 92 wt.%, 93 wt.%, 94 wt.%, 95 wt.%, 96 wt.%, 97 wt.%, 98 wt.%, 98 wt.%, and 100 wt.% of MTBE.

In FIGS. 1-3 the reactors, units and/or zones can include one or more heating and/or cooling devices (*e.g*., insulation, electrical heaters, jacketed heat exchangers in the wall) and/or controllers (*e.g.,* computers, flow valves, automated values, *etc.*) that can be used to control the reaction temperature and pressure of the reaction mixture. While only one unit or zone is shown, it should be understood that multiple reactors or zones can be housed in one unit or a plurality of reactors housed in one heat transfer unit. In some aspects, the reactor can be a fixed bed reactor, moving bed, trickle-bed reactor, rotating bed reactor, slurry reactors or fluidized bed reactor.

In the context of the present invention, at least the following 20 embodiments are described. Embodiment 1 is directed to a process for producing methyl tertiary butyl ether (MTBE), the process comprising: (a) contacting an isobutylene feed with a first stream comprising methanol under conditions suitable to obtain a second stream comprising MTBE and unreacted isobutylene; (b) separating the second stream to obtain a third stream comprising isobutylene and a products stream comprising MTBE; (c) contacting the third stream with a fourth stream comprising methanol under conditions suitable to obtain a fifth stream comprising MTBE; and (d) separating the fifth stream to obtain a sixth stream comprising MTBE. Embodiment 2 is directed to the process of embodiment 1, wherein the second stream is separated in a first MTBE separation unit and the process further comprises feeding the sixth stream to the first MTBE separation unit. Embodiment 3 is directed to the process of any one of embodiments 1 or 2, wherein the contacting condition of the isobutylene feed with the first stream and/or contacting condition of the third stream with the fourth stream comprises a pressure of 10 bar to 25 bar, preferably 15 bar to 20 bar, and/or a temperature of 35 °C to 90 °C, preferably 40 °C to 70 °C. Embodiment 4 is directed to the process of any one of embodiments 1 to 3, wherein the isobutylene conversion in step (a) and/or step (c) is 70 to 95%. Embodiment 5 is directed to the process of any one of embodiments 1 to 4, wherein the products stream comprises 95 wt. % to 100 wt. % of MTBE. Embodiment 6 is directed to the process of any one of embodiments 1 to 5, wherein a seventh stream comprising isobutane and isobutylene from a dehydrogenation unit is separated to obtain a eighth stream comprising isobutylene and a ninth stream comprising isobutane, and the eight stream is fed to step (a) forming the isobutylene feed for step (a). Embodiment 7 is directed to the process of embodiment 6, wherein the seventh stream comprises 30 to 50 wt.% of isobutylene and 30 to 50 wt.% of isobutane. Embodiment 8 is directed to the process of any one of embodiments 6 or 7, wherein the eighth stream comprises 60 to 95 wt.% of isobutylene and 5 to 30 wt.% of isobutane. Embodiment 9 is directed to the process of any one of embodiments 6 to 8, wherein the ninth stream comprises 1 to 35 wt.% of isobutylene and 55 to 99 wt.% of isobutane. Embodiment 10 is directed to the process of any one of embodiments 6 to 9, wherein the ninth stream is recycled to the dehydrogenation unit. Embodiment 11 is directed to the process of any one of embodiments 1 to 5, wherein a raffinate-1 stream comprising isobutylene is fed to step (a), forming the isobutylene feed for step (a). Embodiment 12 is directed to the process of embodiment 11, wherein the raffinate-1 stream comprises 30 wt.% to 60 wt.% of isobutylene, 5 wt.% to 10 wt.% of isobutane, 15 wt.% to 30 wt.% of 1-butene, 0.1 wt.% to 10 wt.% of trans-butene, 0.1 wt.% to 10 wt.% of n-butane, 0.1 wt.% to 5 wt.% of cis-butene, and 0 to 5 wt.% of 1, 3 butadiene. Embodiment 13 is directed to the process of any one of embodiments 11 or 12, wherein in step (d) the fifth stream is separated to form the sixth stream and an eleventh stream comprising n-butane, isobutane, 1-butene, cis-butene, trans-butene, or 1, 3 butadiene or any combination thereof, and the process further comprises separating the eleventh stream to obtain an twelfth stream comprising iso-butane and/or n-butane and a thirteenth stream comprising 1-butene, cis-butene, trans-butene, and/or 1, 3 butadiene. Embodiment 14 is directed to a system for producing MTBE, the system comprising: a first reactor configured to receive an isobutylene feed and a first stream comprising methanol, and produce a second stream comprising MTBE and unreacted isobutylene by a reaction of methanol and isobutylene; a first MTBE separation unit operatively connected to the first reactor and arranged downstream to the first reactor, configured to receive the second stream, separate MTBE and isobutylene, and produce a products stream comprising MTBE and a third stream comprising isobutylene; a second reactor operatively connected to the first MTBE separation unit and arranged downstream to the first MTBE separation unit, configured to receive the third stream and a fourth stream comprising methanol and produce a fifth stream comprising MTBE by a reaction of methanol and isobutylene; and a second MTBE separation unit operatively connected to the second reactor and arranged downstream to the second reactor, configured to receive the fifth stream, separate MTBE from C4 hydrocarbons, and produce a sixth stream comprising MTBE. Embodiment 15 is directed to the system of embodiment 14, wherein a volume of the first reactor is larger than a volume of the second reactor. Embodiment 16 is directed to the system of any one of embodiments 14 or 15, further comprising a C4 separation unit configured to separate C4 hydrocarbons, wherein the C4 separation unit is operatively connected to the first reactor and/or the second MTBE separation unit. Embodiment 17 is directed to the system of embodiment 16, wherein the C4 separation unit is operatively connected to the first reactor and is configured to receive a seventh stream comprising isobutane and isobutylene, separate at least a portion of the isobutane and isobutylene to produce an eight stream comprising isobutylene and a ninth stream comprising isobutane, wherein the eight stream is fed to the first reactor forming the isobutylene feed of the first reactor. Embodiment 18 is directed to the system of any one of embodiments 16 or 17, wherein the system further comprises a dehydrogenation unit, configured to dehydrogenate isobutane to form isobutylene, and the seventh stream is obtained from the dehydrogenation unit. Embodiment 19 is directed to the system of embodiment 18, wherein the ninth stream is recycled to the dehydrogenation unit. Embodiment 20 is directed to the system of embodiment 17, wherein a raffinate-1 stream is fed to the first reactor forming the isobutylene feed for the first reactor, and the C4 separation unit is operatively connected to the second MTBE separation unit and, wherein a eleventh stream containing C4 hydrocarbons is obtained from the second MTBE separation unit and the eleventh stream is separated in the C4 separation unit to form a twelfth stream containing n-butane and iso-butane and thirteenth stream containing 1-butene, cis-butene, trans-butene or 1, 3-butadiene or any combination thereof.

### EXAMPLES

The present invention will be described in greater detail by way of specific examples. The following examples are offered for illustrative purposes only, and are not intended to limit the invention in any manner. Those of skill in the art will readily recognize a variety of noncritical parameters which can be changed or modified to yield essentially the same results.

### Example 1

### Process for producing MTBE from C4 dehydrogenation feed

MTBE was produced using a feed from a dehydrogenation unit. A first cut model was built in Aspen-Plus VlO Software. Simulations were performed based on an embodiment of the current disclosure as shown in FIG. 2. A C-4 feed stream from a C4 dehydrogenation unit was separated in a C4 separation unit to form an isobutylene containing stream and an isobutane containing stream. The isobutylene containing stream was contacted with a first methanol stream in a first reactor to form a crude product stream containing MTBE and unreacted isobutylene. The crude product stream was separated in a first MTBE separator to form a products stream containing MTBE as the bottom product and an stream containing unreacted isobutylene as the top product. The stream containing unreacted isobutylene was contacted with a second methanol stream in a second reactor to form a second crude product stream containing MTBE. The second crude product stream was separated in a second MTBE separator to form a MTBE containing stream as the bottom product and a hydrocarbons stream as top product. The MTBE containing stream was recycled to the first MTBE separator. Tables 1-7 provide the composition of the various streams.

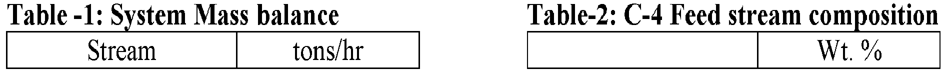

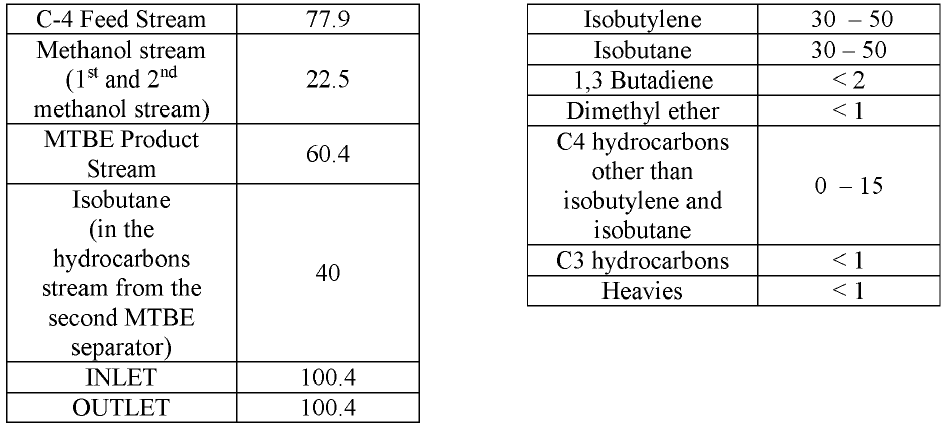

**Table-3: 1st and 2nd Methanol Stream**

| | Wt. % |
|---|---|
| Methanol | > 99 |
| Water | < 0.5 |
| Dimethyl ether | < 1 |

**Table-4: MTBE Products Stream**

| | Wt. % |
|---|---|
| MTBE | > 98 |

**Table-5: Top product from the first MTBE seoaration unit**

| | Wt. % |
|---|---|
| Isobutylene | 20 - 30 |
| Isobutane | 20 - 60 |
| Propane | 0-0.5 |
| MTBE | 0-20 |
| Methanol | 0 - 20 |

**Table-6: Top product from the second MTBE separation unit**

| | Wt. % |
|---|---|
| Isobutane | 95-99 |
| Isobutylene | < 3 |
| Propane | < 2 |

### Example 2

### Process for producing MTBE from a raffinate-1 feed

MTBE was produced using a raffinate-1 feed. A first cut model was built in Aspen-Plus VlO Software. Simulations were performed based on an embodiment of the current disclosure as shown in FIG. 3. A raffinate-1 stream was contacted with a first methanol stream in a first reactor to form a crude product stream containing MTBE and unreacted isobutylene. The crude product stream was separated in a first MTBE separator to form a products stream containing MTBE as the bottom product and an stream containing unreacted isobutylene as the top product. The stream containing unreacted isobutylene was contacted with a second methanol stream in a second reactor to form a second crude product stream containing MTBE. The second crude product stream was separated in a second MTBE separator to form a MTBE containing stream as bottom product and a hydrocarbons stream as top product. The MTBE containing stream was recycled to the first MTBE separator. The hydrocarbons stream was separated in a C4 separation unit to form a butanes stream as a top product and butenes stream as a bottom product. The butenes stream was further separated in a butene separation column to form a stream containing 1-butene as top product and a stream containing cis-butene, trans-butene and 1,3 butadiene as a bottom product. Tables 8-12 provide the composition of the various streams.

**Table -8: System Mass balance**

| Stream | tons/hr |
|---|---|
| C-4 raffinate -1 Feed Stream | 28 |
| Methanol stream (1^{st} and 2^{nd} methanol stream) | 7.5 |
| MTBE Product Stream | 22.5 |
| C-4 hydrocarbons stream (Top product from the second MTBE separator) | 13 |
| INLET | 35.5 |
| OUTLET | 35.5 |

**Table-9: C-4 Raffinate-1 stream composition**

| | Wt. % |
|---|---|
| Isobutylene | 30 - 50 |
| Isobutane | 5 - 10 |
| 1-Butene | 15-30 |
| cis-2-butene | < 5 |
| trans-2-butene | < 10 |
| n-butane | < 10 |
| 1,3 butadiene | < 5 |

**Table-10: 1st and 2nd Methanol Stream**

| | Wt. % |
|---|---|
| Methanol | > 99 |
| Water | < 0.5 |
| Dimethyl ether | < 1 |

**Table-11: Top product from the first MTBE separation unit**

| | Wt. % |
|---|---|
| Isobutylene | 5 - 10 |
| Isobutane | 10 - 15 |
| 1-Butene | 25 - 50 |
| cis-2-butene | < 10 |
| trans-2-butene | < 15 |
| n-butane | < 5 |
| 1,3 butadiene | < 5 |

**Table-12: Top product from the butene separation unit**

| | Wt. % |
|---|---|
| 1-Butene | >98 % |
| n-butane | < 1 |
| Iso-butylene | < 1 |

The operational conditions for the first reactor, second reactor, 1st MTBE separator, 2nd MTBE separator, and the C4 separation unit was kept same for Examples 1 and 2. Thus, the system and process of the current invention shows operational flexibility and can handle different isobutylene feed stock, without the need for a major reconfiguration of the system units.

Although embodiments of the present application and their advantages have been described in detail, it should be understood that various changes, substitutions and alterations can be made herein without departing from the spirit and scope of the embodiments as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the above disclosure, processes, machines, manufacture, compositions of matter, means, methods, or steps, presently existing or later to be developed that perform substantially the same function or achieve substantially the same result as the corresponding embodiments described herein can be utilized. Accordingly, the appended claims are intended to include within their scope such processes, machines, manufacture, compositions of matter, means, methods, or steps.

## Claims

1. A process for producing methyl tertiary butyl ether (MTBE), the process comprising:
(a) contacting an isobutylene feed with a first stream comprising methanol under conditions suitable to obtain a second stream comprising MTBE and unreacted isobutylene;
(b) separating the second stream to obtain a third stream comprising isobutylene and a products stream comprising MTBE;
(c) contacting the third stream with a fourth stream comprising methanol under conditions suitable to obtain a fifth stream comprising MTBE; and
(d) separating the fifth stream to obtain a sixth stream comprising MTBE.

2. The process of claim 1, wherein the second stream is separated in a first MTBE separation unit and the process further comprises feeding the sixth stream to the first MTBE separation unit.

3. The process of any one of claims 1 or 2, wherein the contacting condition of the isobutylene feed with the first stream and/or contacting condition of the third stream with the fourth stream comprises a pressure of 10 bar to 25 bar, preferably 15 bar to 20 bar, and/or a temperature of 35 °C to 90 °C, preferably 40 °C to 70 °C.

4. The process of any one of claims 1 to 3, wherein the isobutylene conversion in step (a) and/or step (c) is 70 to 95%.

5. The process of any one of claims 1 to 4, wherein the products stream comprises 95 wt. % to 100 wt. % of MTBE.

6. The process of any one of claims 1 to 5, wherein a seventh stream comprising isobutane and isobutylene from a dehydrogenation unit is separated to obtain a eighth stream comprising isobutylene and a ninth stream comprising isobutane, and the eight stream is fed to step (a) forming the isobutylene feed for step (a).

7. The process of claim 6, wherein the seventh stream comprises 30 to 50 wt.% of isobutylene and 30 to 50 wt.% of isobutane.

8. The process of any one of claims 6 or 7, wherein the eighth stream comprises 60 to 95 wt.% of isobutylene and 5 to 30 wt.% of isobutane.

9. The process of any one of claims 6 to 8, wherein the ninth stream comprises 1 to 35 wt.% of isobutylene and 55 to 99 wt.% of isobutane.

10. The process of any one of claims 6 to 9, wherein the ninth stream is recycled to the dehydrogenation unit.

11. The process of any one of claims 1 to 5, wherein a raffinate-1 stream comprising isobutylene is fed to step (a), forming the isobutylene feed for step (a).

12. The process of claim 11, wherein the raffinate-1 stream comprises 30 wt.% to 60 wt.% of isobutylene, 5 wt.% to 10 wt.% of isobutane, 15 wt.% to 30 wt.% of 1-butene, 0.1 wt.% to 10 wt.% of trans-butene, 0.1 wt.% to 10 wt.% of n-butane, 0.1 wt.% to 5 wt.% of cis-butene, and 0 to 5 wt.% of 1, 3 butadiene.

13. The process of any one of claims 11 or 12, wherein in step (d) the fifth stream is separated to form the sixth stream and an eleventh stream comprising n-butane, isobutane, 1-butene, cis-butene, trans-butene, or 1, 3 butadiene or any combination thereof, and the process further comprises separating the eleventh stream to obtain an twelfth stream comprising iso-butane and/or n-butane and a thirteenth stream comprising 1-butene, cis-butene, trans-butene, and/or 1, 3 butadiene.

14. A system for producing MTBE, the system comprising:
a first reactor configured to receive an isobutylene feed and a first stream comprising methanol, and produce a second stream comprising MTBE and unreacted isobutylene by a reaction of methanol and isobutylene;
a first MTBE separation unit operatively connected to the first reactor and arranged downstream to the first reactor, configured to receive the second stream, separate MTBE and isobutylene, and produce a products stream comprising MTBE and a third stream comprising isobutylene;
a second reactor operatively connected to the first MTBE separation unit and arranged downstream to the first MTBE separation unit, configured to receive the third stream and a fourth stream comprising methanol and produce a fifth stream comprising MTBE by a reaction of methanol and isobutylene; and
a second MTBE separation unit operatively connected to the second reactor and arranged downstream to the second reactor, configured to receive the fifth stream, separate MTBE from C4 hydrocarbons, and produce a sixth stream comprising MTBE.

15. The system of claim 14, wherein a volume of the first reactor is larger than a volume of the second reactor.
